# EUROPÄISCHE PATENTSCHRIFT

(11) **EP 3 417 937 B1**
(45) Veröffentlichungstag und Bekanntmachung des Hinweises auf die Patenterteilung: **14.04.2021**
(21) Anmeldenummer: 18176248.5
(22) Anmeldetag: 06.06.2018
(51) Int. Cl.: B01J 20/32, B01J 20/24, B01J 20/26, B01J 20/28, B01J 20/30

(54) **HÄMOKOMPATIBLER ADSORBER ZUR DIALYSE PROTEINGEBUNDENER URÄMIETOXINE**
HEMOCOMPATIBLE ADSORBER FOR DIALYSIS OF PROTEIN-BOUND UREMIC TOXINS
ADSORBANT HÉMOCOMPATIBLE DESTINÉE À LA DIALYSE DES TOXINES URÉMIQUES LIÉES AUX PROTÉINES

(30) Priorität: 22.06.2017 DE 102017113853
(43) Veröffentlichungstag der Anmeldung: 26.12.2018
(73) Patentinhaber: B. Braun Avitum AG, 34212 Melsungen (DE)
(72) Erfinder: Baier-Goschütz, Dr. Angela, 01816 Bad Gottleuba-Berggießhübel (DE); Friebe, Dr. Alexander, 45663 Recklinghausen (DE); Napierala, Roland, 33824 Werther (DE); Gäbler, Juliane, 01896 Pulsnitz (DE)
(74) Vertreter: Winter, Brandl - Partnerschaft mbB

(56) Entgegenhaltungen:
- EP-A1- 2 918 337
- WO-A1-2007/013122
- WO-A2-2015/091842
- PIN CHEN ET AL: "Development of a Layer-by-Layer Assembled Film on Hydrogel for Ocular Drug Delivery", INTERNATIONAL JOURNAL OF POLYMER SCIENCE, Bd. 2015, 1. Januar 2015 (2015-01-01), Seiten 1-9, XP055516651, ISSN: 1687-9422, DOI: 10.1155/2015/535092
- JATUPOL JUNTHIP ET AL: "Layer-by-layer coating of textile with two oppositely charged cyclodextrin polyelectrolytes for extended drug delivery : INTRISIC ANTIBACTERIAL ACTIVITY OF MULTILAYER ASSEMBLIES", JOURNAL OF BIOMEDICAL MATERIALS RESEARCH. PART A, Bd. 104, Nr. 6, 2. Februar 2016 (2016-02-02), Seiten 1408-1424, XP055516674, HOBOKEN, NY, US ISSN: 1549-3296, DOI: 10.1002/jbm.a.35674
- BERNARD MARTEL ET AL: "Grafting of cyclodextrins onto polypropylene nonwoven fabrics for the manufacture of reactive filters. III. Study of the sorption properties", JOURNAL OF APPLIED POLYMER SCIENCE, Bd. 85, Nr. 8, 22. August 2002 (2002-08-22), Seiten 1771-1778, XP055516858, ISSN: 0021-8995, DOI: 10.1002/app.10682
- ZHAO X-B ET AL: "Synthesis and characterization of polymer-immobilized beta-cyclodextrin with an inclusion recognition functionality", REACTIVE POLYMERS, ELSEVIER SCIENCE PUBLISHERS, AMSTERDAM, NL, Bd. 24, Nr. 1, 1. November 1994 (1994-11-01), Seiten 9-16, XP024184170, ISSN: 0923-1137, DOI: 10.1016/0923-1137(94)90130-9 [gefunden am 1994-11-01]

## Beschreibung

Die vorliegende Erfindung betrifft einen hämokompatiblen Adsorber gemäß Anspruch 1 sowie eine Vorrichtung zur Hämodiafiltration gemäß Anspruch 6.

Aus der Physiologie der Niere ist es allgemein bekannt, dass es die Aufgabe einer gesunden Niere ist, Endprodukte des Stoffwechsels als sogenannte "harnpflichtige Substanzen" und Giftstoffe, sogenannte "Urämietoxine" aus dem Körper über den Harn auszuscheiden. Die Niere entfernt dabei ein breites Spektrum an Substanzen unterschiedlicher Molekularmassen. Eine Übersicht über urämische Toxine wurde von 2003 von Vanholder et al. veröffentlicht. [vgl. Vanholder et al., Kidney International,63(2003) 1934 - 1943] Die urämischen Toxine werden an Hand ihrer Molekularmassen im Wesentlichen in drei Klassen eingeteilt:
A) Toxine mit niedriger Molekularmasse, welche eine Molekularmasse von < 500 g/mol aufweisen;
B) Toxine mit einer mittleren Molekularmasse, auch "Mittelmoleküle" genannt, welche eine Molekularmasse zwischen 500 und 12 000 g/mol aufweisen. Zu den Mittelmolekülen gehört beispielsweise β2-Microglobulin (11 800 g/mol).
C) Die dritte Klasse von Urämietoxinen bilden Moleküle mit einer Molekularmasse von > 12 000 g/mol.

Darüber hinaus wird nach der Wasserlöslichkeit der Urämietoxine unterschieden. Beispiele für gut wasserlösliche urämische Toxine mit einer niedrigen Molekularmasse sind Harnstoff, Creatinin, Oxalate, Guanidine und Harnsäure.

Beispiele für schlecht wasserlösliche urämische Toxine sind p-Kresol, Indoxylsulfat, Phenol, Hippursäure und Homocystein. Diese Urämietoxine liegen im Serum überwiegend an Proteine gebunden vor.

Bei gesunden Personen werden die Urämietoxine über die Niere mit dem Harn ausgeschieden. Beim chronischen Nierenversagen verbleiben diese Toxine jedoch im Blut des Patienten und müssen durch Hämodialyse oder Peritonealdialyse entfernt werden.

Während die Entfernung wasserlöslicher Toxine wie beispielsweise Harnstoff oder Creatinin mittels Hämodialyse sehr gut möglich ist, ist die Entfernung von schlecht wasserlöslichen hydrophoben Urämietoxinen mittels Hämodialyse durch die Proteinbindung extrem erschwert, da proteingebundene Urämietoxine nur über das chemische Gleichgewicht des Toxin-Protein-Komplexes mit freiem Toxin im Blutplasma der Hämodialyse zugänglich ist, wobei das Gleichgewicht stark auf der Seite des Komplexes liegt. Dies bedeutet, dass der überwiegende Teil der Urämietoxine an Proteine gebunden ist, während nur ein kleiner Teil im Blutplasma gelöst ist und damit nur diese frei vorliegenden Urämietoxine dialysierbar sind.

Weitere Untersuchungen zur physiologischen Chemie der proteingebundenen Urämietoxine haben ergeben, dass humanes Serumalbumin als Bindungspartner der hydrophoben Urämietoxine fungiert und sich somit Toxin-Albumin-Komplexe im Blut des Patienten bilden.

Albumin wird aufgrund seiner Molekularmasse von ca. 65 000 g/mol von gängigen Dialysemembranen zurückgehalten. Albumin wird daher durch Hämodialyse nicht entfernt. Damit kann nur der freie, gelöste und sehr geringe Anteil der urämischen Toxine aus dem Blut des Patienten entfernt werden. Nach der Entfernung dieses geringen freien Anteils stellt sich das Gleichgewicht zwischen albumingebundenen und freien Urämietoxinen erneut ein.

Theoretisch könnte man über diese Neueinstellung des Gleichgewichts einen beträchtlichen Teil der freien Urämietoxine durch kontinuierliche Dialyse entfernen. Dem stehen jedoch zu große Assoziationskonstanten des Toxin-Albumin-Komplexes sowie eine nicht hinreichende praktikable Dialysezeit entgegen.

Es besteht somit seit langem ein Bedarf an Dialyseverfahren, die in der Lage sind, auch proteingebundene Urämietoxine aus dem Blut eines Patienten mit chronischem Nierenversagen oder chronischer hochgradiger Niereninsuffizienz wirksam zu entfernen.

Hierzu gibt es bereits eine Reihe von Ansätzen im Stand der Technik, von denen sich aus unterschiedlichen Gründen bislang jedoch keiner im klinischen Alltag durchgesetzt hat:
Die GB 1 466 702 offenbart polymerbeschichtete Aktivkohle zur Adsorption urämischer Toxine im Verdauungstrakt.

Eine Verwendung von polymerbeschichtete Aktivkohle zur Adsorption urämischer Toxine aus Blut ist aus der US 4,140,652 bekannt.

Die WO 2009/157877 offenbart ein Verfahren zur Entfernung von Toxinen aus einer Dialysierflüssigkeit durch Adsorption an ZrO₂-Partikel.

Ferner offenbart die WO 2010/045474 ein kompetitives Verfahren zum Abtrennen proteingebundener urämischer Toxine durch Hinzugeben einer Substanz, welche die Toxine von ihren Bindungsstellen am Protein verdrängt und diese damit der Dialyse zugänglich macht.

Die US 4,889,634 offenbart ferner eine Hydroxypropyl-β-Cyclodextrin enthaltende Lösung für die Peritonealdialyse.

Die WO 2007/013122 offenbart die Verwendung von Hämofiltern zur Entfernung von bakteriellen Toxinen (Lipopolysacchariden) aus dem Blut, wobei die Hämofilter einen festen Träger aufweisen, an den Cyclodextrine kovalent gebunden sind.

Zusammenfassend kann somit festgehalten werden, dass die Einstellung des Gleichgewichtes zwischen proteingebundenen, insbesondere albumingebundenen Urämietoxinen und frei vorliegenden Urämietoxinen unter Dialysebedingungen der geschwindigkeitsbestimmende Schritt ist. Wie oben erwähnt, ist es zwar zu erwarten, dass sich nach der Entfernung der gelösten Toxine aus dem Blut das Gleichgewicht zwischen freien und proteingebundenen Toxinen wieder neu einstellt und bei genügend langer Dialysezeit ein beträchtlicher Teil der Toxine entfernen lässt, diese Zeit steht bei Hämodialysebehandlungen jedoch nicht zur Verfügung.

Während einer Dialyse wird Albumin aufgrund seiner Molekularmasse von den üblichen Dialysemembranen zurückgehalten und damit auch die daran gebundenen Toxine.

Überführt man jedoch gemäß WO2015091842 A2 [15] die proteingebundenen Urämietoxine auf einen niedermolekularen Bindungspartner (statt des hochmolekularen Proteinpartners), so dass der resultierende Komplex aus Bindungspartner und Urämietoxin eine Molekularmasse aufweist, die im dialysierbaren Bereich liegt, kann beim Durchgang durch den Dialysator sowohl das in freier Lösung vorliegende Toxin als auch das zuvor in Albuminbindung vorliegende Toxin entfernt werden. Derartige niedermolekulare Bindungspartner werden im Rahmen der Offenbarung der [15] als dialysierbare Hilfsstoffe bei Dialysen zugesetzt.

Gemäß [15] wird insbesondere vorgeschlagen, einen dialysierbaren Hilfsstoff beispielsweise blutseitig in den Zulaufschlauch eines Dialysators einzudosieren, so dass sich auf dem Weg zum Dialysator das gewünschte Gleichgewicht zwischen Bindungspartner und Toxin ausbildet. Dabei wird das Toxin aus seinen Bindungsstellen am Albumin oder an weiteren Proteinen entfernt. Der Bindungspartner mit dem gebundenen Toxin kann sodann effizient durch ein herkömmliches Dialyseverfahren entfernt werden. Als derartige kompetitive Hilfsstoffe schlägt die WO2015091842 A2 vor, unter anderen Cyclodextrine einzusetzen, welche Molekularmassen im Bereich von 500 bis 5000 g/mol, vorzugsweise 1000 bis 2000 g/mol, aufweisen.

Die Substanzklasse der Cyclodextrine ist für die Bindung von hydrophoben Verbindungen bekannt.

Cyclodextrine sind eine Klasse von Verbindungen, die zu den cyclischen Oligosacchariden gehören. Sie stellen ringförmige Abbauprodukte von Stärke dar. Sie bestehen aus α-1,4-glykosidisch verknüpften Glucosemolekülen. Dadurch entsteht eine toroidale Struktur mit einem zentralen Hohlraum. Je nach Anzahl der verknüpften Glucosemoleküle erhalten sie einen griechischen Buchstaben als Präfix:
Die Eigenschaften von Cyclodextrinen wie die Wasserlöslichkeit lassen sich durch Substitution der Hydroxylgruppen beeinflussen. So erhöht sich beispielsweise die Wasserlöslichkeit von β-Cyclodextrin durch Methylsubstitution um den Faktor 150. Darüber hinaus kann durch gezielte Substitution die Adsorptionseigenschaften der Cyclodextrine verändert werden. Beispielsweise darf β-Cyclodextrin nicht intravenös verabreicht werden, da es unlösliche Komplexe mit Cholesterin bildet. Die substituierten Cyclodextrine Hydroxypropyl-β-Cyclodextrin (HPBCD) und Sulfobutylether-β-cyclodextrin (SBECD) bilden keine unlöslichen Cholesterinkomplexe und sind damit bevorzugte Cyclodextrine zur Verwendung gemäß der [15].

Neben den oben genannten Cyclodextrinverbindungen gibt es weitere Derivate der verschiedenen Cyclodextringrundkörper, die für die erfindungsgemäße Anwendung in Frage kommen. Sie sind dem Fachmann aus Übersichtsartikeln wie beispielsweise dem von Brewster et. al. (Advanced Drug Delivery Reviews 59 (2007) 645-666) bekannt und zeichnen sich in aller Regel durch die Einführung hydrophiler Gruppen an substituierbaren Gruppen des Cyclodextrinmoleküls aus.

Prinzipiell kommen neben den Cyclodextrinen auch andere makrozyklische Systeme in Frage, die vergleichbare strukturelle Eigenschaften aufweisen. Eine Übersicht dazu gibt der Review von Biros et. al.. (Chem. Soc. Rev., 2007,36, 93-104) Dort sind neben den Cyclodextrinen auch Calix[n]arene, Cyclophane und Curcubiturilderivate beschrieben.

Gemäß [15] ist der dialysierbare Hilfsstoff in einer Dialyse- oder Substitutionslösung gelöst. In einer weiteren Ausführungsform kann der dialysierbare Hilfsstoff in einer physikalischen Mischung mit Salzen, wie beispielsweise Natriumchlorid, vorliegen. Zur Herstellung einer gebrauchsfertigen Lösung wird die Mischung in Wasser zur Injektion, gegebenenfalls unter Zusatz weiterer Elektrolyte aufgelöst. Eine solche gebrauchsfertige Lösung eignet sich gemäß WO2015091842 A2 auch als Infusionslösung.

Die Lehre der [15] betrifft weiterhin eine Vorrichtung und ein Verfahren zur Entfernung proteingebundener Toxine durch Zugabe eines dialysierbaren Hilfsstoffs.

Die Lehre der [15] betrifft insbesondere eine Vorrichtung zur Durchführung des beschriebenen Verfahrens zur Entfernung proteingebundener Toxine umfassend einen extrakorporalen Kreislauf zur Aufnahme von zu reinigendem Blut sowie mit einem Hämodialysator und/oder Hämofilter, der mit dem Blutkreislauf in Verbindung steht, wobei der Blutkreislauf stromaufwärts und optional stromabwärts von dem Hämodialysator und/oder Hämofilter jeweils mindestens eine Zuleitung für die Zufuhr einer Substitutionsflüssigkeit aufweist. Die stromaufwärts von dem Hämodialysator und/oder Hämofilter über eine Zuleitung zugeführte Substitutionsflüssigkeit umfasst dabei den dialysierbaren Hilfsstoff.

Für die Elimination von urämischen Toxinen mittels Hämodialyse und Hämo(dia)filtration stehen heute unterschiedliche Membranen zur Verfügung. Um eine Effiziente Dialysebehandlung sicherzustellen, spielt die Membraneigenschaft eine wesentliche Rolle. [vgl. 1-5] Eine Beurteilung über die Effizienz einer Dialysebehandlung kann durch die Bestimmung der Eliminationskapazität für kleinmolekulare Substanzen [2,4,5], meist wasserlöslich und nicht proteingebunden [vgl. 5-6] erfolgen. Die jüngsten klinischen Publikationen weisen jedoch darauf hin, dass die biochemischen Veränderungen in einer Urämie nicht nur von wasserlöslich/nicht proteingebunden Toxinen ausgelöst werden [4], sondern auch von proteingebundenen Substanzen/Toxinen, wie p-Kresol, Indoxylsulfat, Hippursäure, 3-carboxy-4-methyl-5-propyl-2-furanpropionsäure (CMPF) oder Phenolderivaten [2,6]. Diese Toxine sind hauptsächlich an Albumin gebunden.

Heute sind p-Kresol und Indoxylsulphat die am häufigsten untersuchten proteingebundenen Toxine bei Dialysepatienten. Hierbei kann weder die rein diffusive (HD), rein konvektive (HF) oder diffusive/konvektive (HDF) Dialyse eine effektive Elimination gewährleisten. Dies liegt hauptsächlich daran, dass - wie oben erwähnt - natürlich die Retention von essenziellen Proteinen wie Albumin auch berücksichtigt werden muss, und die Membranen zur normalen Anwendung bei Dialysepatienten diese Selektivität nicht bieten. Weil aber p-Kresol, Indoxylsulfat und weitere proteingebundene Toxine eine hohe kardiovaskuläre Toxizität aufweisen, stellt es bis in die heutigen Tage ein Problem für die Dialyseanwendung dar.

In der Literatur findet man verschiedene Arbeiten in denen beschrieben wird, wie aus Blut, humanem Plasma oder aus einer plasmaimitierenden Lösung mehr oder wenige selektiv urämische Toxine verschiedener Molekülgröße adsorbiert werden können.

### Adsorption basierend auf Aktivkohle

Ein frühes Beispiel für eine medizinisch verwendbare Aktivkohle mit einem Copolymer, welches aus Acrylsäure und Styrol besteht und ein blutkompatibles Coating ergibt, wurde im Jahr 1974 von Sparks et al. (Sandoz AG) patentiert [7, DD 109 363]. Diese von einem Polymer umschlossene Aktivkohle ist in der Lage, ungeladene niedermolekulare urämische Toxine (z.B. Harnstoff oder Kreatinin) zu adsorbieren. Im Jahre 2015 patentierte Tschulena et al. (Fresenius AG) eines der bisher vorteilhaftesten Adsorber-Granulate [8]. Hierzu wird aus einer wässrigen Lösung aus Polyvinylpyrrolidon (PVP), einem zusätzlichen Vernetzer und einem Radikalinitiator eine Aktivkohle, bestehend aus sphärischen Partikeln unter Erhitzen "gepfropft"; d.h. das Polymer wird fest auf der Aktivkohle angebunden und bildet zusammen mit dem Vernetzer ungeladene, mesoporöse Strukturen, durch die hydrophobe und geladene, niedermolekulare urämische Toxine diffundieren können (z.B. Indoxylsulfat, p-Kresolsulfat, Bilirubin; Ap5A und Phenylessigsäure).

Stamatialis et al. publizierten im Jahr 2016 die Entwicklung einer Low-Flux-Hohlfasermembran auf Basis von Polyethersulfon (PES), die aus zwei miteinander kombinierten Schichten besteht - "Mixed-Matrix-Membrane" (MMM). Die innere Schicht für den Blutkontakt besteht aus reinem PES, während die äußere Schicht aus einem Komposit mit fein verteilter Aktivkohle in PES zusammengesetzt ist. Die Gruppe hat gezeigt, dass sich mit Hilfe dieser Membranen Indoxylsulfat oder p-Kresolsulfat (künstlich zugesetzt und an Albumin gebunden) in menschlichem Plasma im Vergleich zu Membranen ohne Aktivkohleschicht mit verdoppelter Effektivität entfernen lässt. Die Effektivität in Bezug zur Entfernung von urämischen Toxinen durch Adsorption an der Aktivkohle addiert sich somit zu der bei einer konventionellen Dialyse stattfindenden Diffusion [9, 10].

In Studien von Glorieux und Krieter et al. [11a und 11b] wurde berichtet, dass das Blutplasma eines gesunden Menschen 0,53 ± 0,29 mg/L Indoxylsulfat aufweist und das eines chronisch nierenkranken Patienten vor einer Dialysebehandlung 44,5 ± 15,3 mg/L [11]. Nach der Dialysebehandlung kann dieser Wert entsprechend einer Studie von Böhringer et al. [11, 12] auf ∼39 mg/L gesenkt werden. Werden die Ergebnisse zu MMM von Stamatialis et al. [9] dazu in Relation gesetzt, so wäre eine MMM in einer Dialysebehandlung in der Lage, diesen Wert auf ∼32 mg/L zu senken.

### Adsorption basierend auf porösem Aryl-Kunstharz

Tlustakova et al. patentierte im Jahr 1984 eines der ersten Beispiele für die Herstellung von einem porösen Adsorber auf Basis von Styrol, Divinylbenzol (DVB) und Acylnitril mit kovalent aufgepfropftem 2-Hydroxyethylmethacrylat incl. einem geeigneten Vernetzer als biokompatible Schicht für den Blutkontakt [13]. Filter solcher Bauart werden bis in die heutigen Tage beispielsweise zur LdL-Apherese benutzt. Auf Grund des hydrophoben Charakters des auf Styrol basierenden Harzes und der zu entfernenden Substanzen sind diese Adsorber besonders wirkungsvoll. In neuerer Zeit stellt z.B. die Firma Cytosorbents Corp. einen Adsorber zur Entfernung von Zytokinen (auch mittelmolekulare Proteine mit hydrophoben Domänen) aus Vollblut her. Der eigentlich für die Adsorption verantwortliche Kern der Partikel ist ebenso auf Basis eines Styrol-DVB-Coplymers, die biokompatible Schutzschicht besteht hierbei aus hochporösem PVP. Somit können sogar Zytokine mit größerem Molekularmasse adsorbiert werden (8 kDa zu 100%, 18 kDa zu 85% und 51 kDa zu 55%) [14].

### Cyclodextrin zur Komplexbildung (Gast- Wirt-Beziehung)

Im Jahre 2013 patentierte Fislage et al. (Fresenius Medical Care Deutschland GmbH) einen bis dahin völlig neuen Ansatz zur Entfernung albumingebundener Toxine mit einer hydrophoben Domäne durch Anwendung des dialysierbaren Hilfsstoffes Cyclodextrin mit einer dafür affinen bindungsfähigen Kavität [15]. Diese Erfindung beruht somit auf einer "Umkomplexierung" in Lösung. Zu einem künstlichen Plasma, hergestellt aus 638 µmol/L an Bovine Serum Albumin (BSA) (entspricht 30 g/L) in Puffer, wurden 202 µmol/L des im Blut nierenkranker Menschen vermehrt vorkommende p-Kresol gegeben. Dies bildet sofort einen Komplex mit dem BSA. Diese Lösung wurde mit einer Flussrate von 100 ml/min. durch die Lumenseite eines handelsüblichen high flux Dialysators (Typ Fresenius FX60) gepumpt. Unmittelbar vor dem Eingang in den Dialysator wurde zudem eine Lösung von Hydroxypropyl-β-cyclodextrin (224 g/L) kontinuierlich mit 10 ml/min. zu dosiert, sodass sich eine Konzentration von 22,4 g/L (16 mmol/L) in der gesamten Lösung einstellte. Am Ausgang des Dialysators (Volumenstrom 110 ml/min.) konnte eine bemerkenswerte Abnahme der Konzentration des p-Kresols auf 55% im Vergleich zur Eingangskonzentration gemessen werden.

Hammond et al. publizierten 2009 weiterhin das erste Beispiel für einen Adsorber auf Basis von Poly(carboxymethyl-β-cyclodextrin) [16]. Dieser anionische Polyelektrolyt wurde in Kombination mit einem biologisch abbaubaren, kationischen Polyelektrolyt als Haftvermittler auf einer Modelloberfläche aus Silizium alternierend durch einen Layer-by-Layer(LbL)-Prozesses immobilisiert. Diese Schichten wurden dazu verwendet, um hydrophobe Substanzen (Antibiotikum, Flurbiprofen und Diclofenac) zu komplexieren. Die schwach negativ geladenen Carboxylgruppen an den Ringen vom Cyclodextrin selbst bilden die Grundlage für die Haftung des Polymers auf dem Substrat.

### Weitere Methoden

Die Bindung zwischen einem Protein und einem der diskutierten hydrophoben Toxine beruht auf der Bildung eines Komplexes, der im wässrigen Milieu des Blutes auf der Seite des Komplexes liegt [11, 12, 17, 18]. Für Indoxylsulfat, Phenylessigsäure und p-Kresolsulfat liegt jeweilig unter physiologischen Bedingungen (NaCl = 0,15 mol/L) 96%, 60% und 97% in der gebundenen Form vor. Wird nun die Salzkonzentration in den hypertonischen Bereich von beispielsweise 0,50 mol/L angehoben, verschiebt sich dieses Gleichgewicht zu 88%, 36% und 91%. Durch den erhöhten Salzgehalt wird die Wechselwirkung zwischen Toxin und Protein gestört. In der Folge kann das niedermolekulare Toxin durch eine Dialysemembran entfernt werden. Diese Vorgehensweise ist bedingt durch physiologische Grenzen problematisch für den Patienten. In derselben Studie untersuchte Böhring et al. die Möglichkeit, die Eliminierung von hydrophoben Toxinen mit Hilfe von hochfrequenten elektromagnetischen Feldern zu beeinflussen, indem die Arbeitsgruppe den Dialysator bei in-vitro Versuchen als Dielektrikum zwischen zwei Kondensatorplatten bzw. als Kern einer Spule genutzt hat. Als Ergebnis wurde festgestellt, dass der Einfluss erheblich keiner ist, als im Falle der Verwendung einer erhöhten Salzkonzentration [11, 12].

### Nachteile bisheriger Lösungsansätze des Standes der Technik

Wird zur Entfernung von hydrophoben, niedermolekularen Bluttoxinen oder Zytokinen eine Aktivkohle oder ein tendenziell hydrophobes Polymer, z.B. ein Copolymerisat aus Styrol und Divinylbenzol (Amberlite XAD-2) als Adsorber verwendet, kann Vollblut nicht direkt mit diesen Materialien in Kontakt gebracht werden. Das ist ein großer Nachteil. Um dies zu umgehen, kann es mit einem Plasmafilter aufgetrennt werden, sodass von festen Blutbestandteilen isoliertes Plasma von einem solchen Adsorber gereinigt und anschließend zurückgeführt werden kann. Bei diesem Verfahren sind jedoch aufwendige Pumpen- und Schlauchsysteme nötig [19]. Eine andere Möglichkeit ist, das Basispolymer mit einem der o.g. biokompatiblen, hydrophilen Polymere zu "maskieren", um einen Kontakt mit Vollblut zu ermöglichen. Für eine solche Funktionalisierung ist in der Regel eine chemische Anknüpfung auf dem Substrat durch sogenannte "radikalische Pfropfcopolymerisation" nötig. Dies zu realisieren ist sehr kostenaufwendig. Die Schutzschicht stellt jedoch eine mehr oder weniger stark ausgeprägte Diffusionsbarriere dar, hauptsächlich für größere Moleküle. Besonders ausgeprägt ist die Barriere im Falle der von Stamatialis et al. vorgeschlagenen MMM. Hierbei trennen ∼40 µm PES die mit p-Kresol beladenen Proteine auf der Blutseite in den beschrieben in-vitro Versuchen von der adsorbierenden Schicht mit der Aktivkohle auf der Dialysatseite [9, 10]. Die hohe Wandstärke der Membranen (∼100 µm) ergeben zudem eine dicke Faser mit einem ungünstigen Verhältnis von Faseranzahl pro Bündeldurmesser. Ein hoher Materialverbrauch für die Herstellung der Membran wird hier ersichtlich und damit wieder ein höherer Kostenfaktor. Konventionelle Adsorber mit Aktivkohle haben weiterhin den Nachteil, dass sie wenig selektiv sind, d.h. viele im Blut vorhandene, auch für die zu behandelnden Patienten lebenswichtige Proteine oder schlecht wasserlösliche Vitamine werden neben schädlichen Molekülen adsorbiert. Ebenso gibt es kein selektives Verhalten im Hinblick auf die Molmasse der zu adsorbierenden Stoffe. Bei der Therapieform der Apherese zur Behandlung von Stoffwechselerkrankungen des Fetthaushaltes (Dyslipidämie) werden diese Effekte zurückgedrängt, da vornehmlich das zu entfernende LDL etc. die Adsorptionsplätze besetzt. Ein weiterer Nachteil bildet generell das große Blutvolumen von 200 bis 400 mL und ein hoher Druckverlust eines solchen Adsorbers. Diese Eigenschaft führt bei den meisten Produkten zu kleinen möglichen Blutflüssen. Diese Eigenschaften führen in Summe dazu, dass eine Inkorporation in ein gewöhnliches System zur Hämodialyse nicht möglich ist.

Ausgehend vom Stand der Technik der WO2015091842 A2 [15] ist es die Aufgabe der vorliegenden Erfindung, die proteingebundenen Toxine vom Albumin zu lösen und diese anschließend effektiv durch Dialyse zu entfernen, ohne zusätzliche Hilfsstoffe in den Patienten und/oder die Dialyseflüssigkeit einzubringen, wie es gemäß der Lehre der [15] erforderlich ist.

Die Lösung dieser Aufgabe erfolgt durch einen hämokompatiblen Adsorber gemäß Patentanspruch 1.

Insbesondere betrifft die vorliegende Erfindung einen hämokompatiblen Adsorber zur Trennung von in Blut oder Blutplasma eines Patienten enthaltenen proteingebundenen Urämietoxinen mit einer Molekularmasse von < 500 g/mol von ihren Trägerproteinen, um die Urämietoxine mittels einer Hämodialyse dialysierbar zu machen, wobei das hämokompatible Material ein Polymer auf Basis eines cyclischen Oligosaccharids oder eines Derivates davon enthält, welches auf einer festen Trägerkomponente in wenigstens einer Schicht angeordnet ist, wie weiter durch Anspruch 1 definiert.

Im Rahmen der vorliegenden Erfindung werden die Urämietoxine bevorzugt ausgewählt aus der Gruppe, bestehend aus p-Kresol, Indoxylsulfat, Phenol, Phenolderivate, Homocystein, Urofuransäuren, insbesondere 3-Carboxy-4-methyl-5-propyl-2-furanpropionsäure, Hippursäure und p-Hydroxyhippursäure.

In einer bevorzugten Ausführungsform ist der erfindungsgemäße Adsorber besonders effektiv, wenn die zu entfernenden Urämietoxine an humanes Albumin als Trägerprotein gebunden sind.

Im Rahmen der vorliegenden Erfindung wird ein hämokompatibler Adsorber eingesetzt, bei welchem das auf cyclischen Oligosacchariden basierende Polymer ausgewählt ist aus der Gruppe, bestehend aus: Poly(carboxymethyl-β-cyclodextrin) und Poly(trimethylammonium-β-cyclodextrin) und mit Epichlorhydrin kondensierten Cyclodextrinen.
Die feste Trägerkomponente ist ein poröses Material auf Basis von Polyethersulfon [PES], Polysulfon [PSU], Polyetheretherketone [PEEK], Polyphenylsulfon [PPSU], Polyoxymethylen [POM], Polyphenol, Polyamide, insbesondere Nylon, Polystyrol, Polycarbonat oder Polymeren, die Acrylnitril oder ein Methylallylsulfonat-Salz oder Copolymere daraus enthalten.

Das Polymer auf Basis eines cyclischen Oligosaccharids oder eines Derivates davon wird typischerweise im Rahmen der vorliegenden Erfindung durch eine Layer-by-Layer-Technik [LbL-Technik] in einer Vielzahl von Schichten auf der festen Trägerkomponente aufgebracht ist. Die LbL-Technik ist dem Fachmann wohlbekannt, beispiesweise aus [16].

Ein besonderer Vorteil des erfindungsgemäßen hämokompatiblen Adsorbers ergibt sich, wenn er als eine, einem Dialysator eines Hämodialysesystems vorgeschaltete separate Kartusche ausgebildet ist, wobei ein darin angeordnetes, insbesondere non-woven, Gewebe mit dem Polymer beschichtet ist.

Ein weiterer besonderer Vorteil des erfindungsgemäßen hämokompatiblen Adsorbers ergibt sich, wenn er als non-woven Gewebe, welches mit dem Polymer beschichtet ist, in eine Blutkappe eines Dialysators eines Hämodialysesystems integriert ist.

Ein im Rahmen der vorliegenden Erfindung beschriebener hämokompatibler Adsorber ist ein solcher, der in das Porensystem der Hohlfasermembranen eines Dialysators eines Hämodialysesystems integriert ist.

Die vorliegende Erfindung betrifft ferner eine Vorrichtung zur Hämodiafiltration mit einem extrakorporalen Kreislauf zur Aufnahme von zu reinigendem Blut sowie mit einem Hämodialysator und/oder einem Hämofilter, der mit dem Blutkreislauf eines Patienten in Verbindung stehen kann;
wobei ein hämokompatibler Adsorber zur Trennung von in Blut oder Blutplasma eines Patienten enthaltenen proteingebundenen Urämietoxinen mit einer Molekularmasse von < 500 g/mol von ihren Trägerproteinen vorgesehen ist; und wobei der hämokompatible Adsorber, welcher blutseitig innerhalb des Hämodialysators auf einer festen Trägerkomponente in wenigstens einer Schicht angeordnet ist, ein Adsorber gemäß der vorliegenden Erfindung ist.

Die vorliegende Erfindung beschreibt somit einen Adsorber für eine selektive und wirksame Entfernung von albumingebundenen Toxinen mit hydrophoben und / oder geladenen Domänen bis zu einem Molgewicht von ∼500 Da (z.B. Indoxylsulfat, p-Kresol, Hippursäure, p-Hydroxyhippursäure oder Phenylessigsäure) zur Unterstützung einer gewöhnlichen extrakorporalen Blutbehandlung (z.B. Hämodialyse oder Leberdialyse).

Der Adsorber besteht aus einem hämokompatiblen Polymer auf Basis von Cyclodextrin, welches mit Hilfe der LbL-Technologie auf ein poröses Material mit einer großen Oberfläche aufgebracht wird. Als Träger eignet sich idealerweise ein non-woven-Gewebe mit passender Maschenweite. Dieses beschichtete Gewebe kann in einer kleinen Kartusche vor dem Dialysator in Reihe geschaltet werden oder in die Blutkappen des Dialysators eingebaut werden. Eine weitere sinnvolle Möglichkeit ist, die gesamte zugängliche Oberfläche einer High-Flux Hohlfasermembran für Hämodialyse mit dem hämokompatiblen Polymer zu beschichten. Das Polymer sollte vorzugsweise zum einen aus dem o.g. von Hammond et al. [16] erstmalig eingeführten Poly(carboxymethyl-β-cyclodextrin) als anionischem Polyelektrolyt bestehen. Zum anderen sollte es dem kationischen Haftvermittler Polyethylenimin (PEI) oder idealerweise einem kationischen Cyclodextrin-Pendant, wie dem neulich von Morin et al. vorgeschlagenen Poly(trimethylammonium-β-cyclodextrin), bestehen [20]. Ein eindeutiger Vorteil des letztgenannten Polyelektrolyten ist, dass es nicht nur wie PEI als Haftvermittler für den LbL-Prozess dient, sondern auch selbst Cyclodextrin enthält. Somit vergrößert sich mit jeder aufgebrachten Schicht die Kapazität des Adsorbers bis zum erwünschten Punkt. Prinzipiell lassen sich diese beiden Polyelektrolyte einfach durch Kondensation von entsprechend modifiziertem Cyclodextrin mit Epichlorhydrin herstellen. Diese Synthesen können in wässrigen Lösungen oder ebenso in organischen Lösemitteln unter einfachen Reaktionsbedingungen durchgeführt werden [21, 22, 23]. Cravotto et al. und Hapiot et al. offenbaren zudem eine neue Möglichkeit für die Modifizierung von Cyclodextrinen als Festphasenreaktion ohne Wasser mit Hilfe einer Kugelmühle [24, 25].

Um einen möglichst guten Übergang des Bluttoxins vom Albumin zum Adsorber zu erreichen, muss die Bindungskonstante des Adsorbers zu dem entsprechenden Toxin groß genug sein. Bei physiologischen Bedingungen (0,15 mol/L NaCl) beträgt dieser Wert zwischen Albumin und Indoxylsulfat Ks = 48.000 L/mol [12]. Im Vergleich dazu besitzt ein in Lösung vorliegendes (natives) Cyclodextrin nur eine Bindungskonstante von Ks = 12.000 L/mol. Entsprechend des Patentes von Fislage et al. konnte jedoch gezeigt werden, dass trotzdem unter den experimentellen Bedingungen ein Großteil von 55% eines Toxins (p-Kresol) entfernt werden kann [15]. Bei einem ungefähren Füllvolumen von 100 mL aller Lumen eines Dialysators mit 1,6 m² effektiver Membranfläche beträgt die Verweilzeit des Modellplasmas bei 110 ml/min ∼55s. Diese Zeit ist offenbar ausreichend, um nach diffusiver Entfernung der ersten "umkomplexierten" Toxine die neue Gleichgewichtseinstellung stattfinden zu lassen und somit eine effektive Entfernung zu erreichen. Der Komplex aus p-Kresol und Hydroxypropyl-β-Cyclodextrin besitzt eine Molmasse von ∼1.500 g/mol und ist somit im Gegensatz zum Albumin-Komplex (ca. 65.200 g/mol) leicht dialysierbar. Zwei weitere Aspekte machen aufgrund dieser Tatsache den im Rahmen der vorliegenden Erfindung vorgeschlagenen Adsorber im Vergleich dazu weitaus effektiver: 1.) das kationische Poly(trimethylammonium-β-cyclodextrin) oder ein mehrfach substituiertes Derivat von diesem enthält einen Liganden, welcher die Bindungskonstante des Adsorbers im Vergleich zu nativem Cyclodextrin um das bis zu 3.000fache (Ks = 12.000 L/mol vs. 3.410.000 L/mol; beides in Lösung) - bedingt durch den quarternären Ammoniumrest - anhebt [18] ; 2.) steigt die Bindungskonstante eines Adsorbers generell um das 10 bis 15fache an, wenn er als Festkörper bzw. quellfähiger Film vorliegt, verglichen mit einem Gast-Wirt-Komplex in Lösung [26]. Der Grund hierfür liegt in der zusätzlich anfallenden physikalisch bedingten Adsorptionsenthalpie am Festkörper. Im Blut chronisch nierenkranker Menschen finden sich neben anderen Substanzen hauptsächlich folgende albumingebunde Toxine: p-Kresol mit 41,0 ± 13,3 mg/L Blut, Indoxylsulfat mit 44,5 ± 15,3 mg/L Blut, Hippursäure mit 87,2 ± 61,7 mg/L Blut und p-Hydroxyhippursäure mit 18,3 ± 6,6 mg/L Blut [11].

Geht man nun davon aus, dass das Blut eines gesunden Menschen verglichen damit nur 1 bis 5 % der Konzentrationen der genannten Stoffe enthält [11], so werden von dem beschriebenen adsorbierend wirkenden Polymer bezogen auf mit Epichlorhydrin vernetztem "nativem" β-Cyclodextrin pro Liter zu reinigendes Blut nur ∼1,4 g trägergebundenes Polymer benötigt.

Es gibt derzeit keinen selektiven Adsorber für proteingebundene Bluttoxine bis ∼500 g/mol. Der erfindungsgemäße Adsorber kann auf jedem beliebigen Substrat mit variierbarer Kapazität und Affinität immobilisiert werden. Er zeichnet sich dadurch aus, dass eine im Vergleich zu anderen Adsorbern nur eine geringe Menge an funktionellem Polymer notwendig ist. Entweder ist für eine Serienschaltung einer separaten Kartusche zu einem Dialysator (Fig. 1) ein kleines zusätzliches Blutvolumen erforderlich, oder es fällt kein zusätzliches Blutvolumen an, wenn es in den Blutkappen des Dialysators (Fig. 2) bzw. auf der gesamten Oberfläche der Hohlfasermembran (Fig. 3) angewendet wird. Weiterhin kommt der Adsorber ohne diffusionshemmende Schutzschicht aus, da das Material selbst für den direkten Blutkontakt geeignet ist.

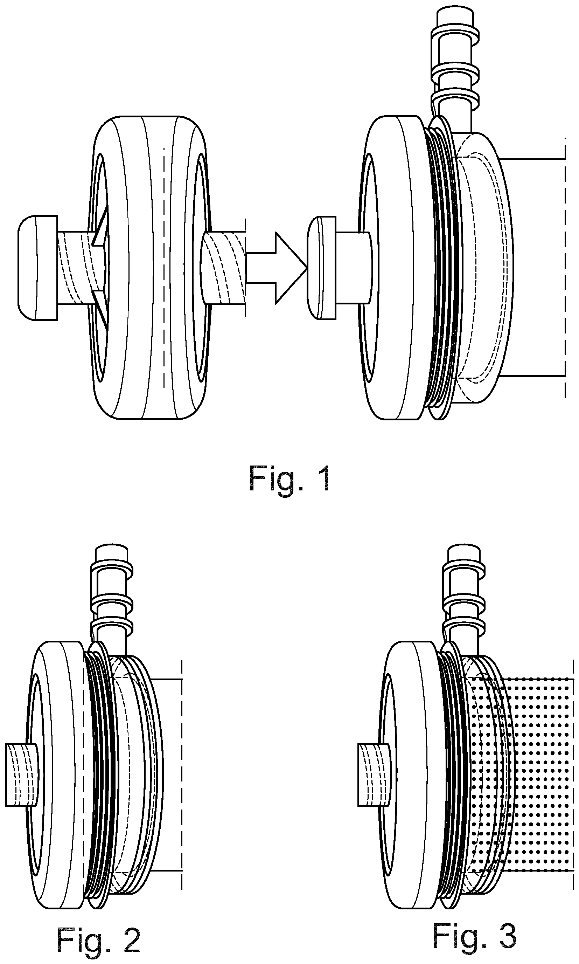

Der erfindungsgemäße Adsorber ist prinzipiell für jede Dialysebehandlung nutzbar, da schlecht dialysierbare Toxine < 500 g/mol wirkungsvoll entfernt werden, die jeden Patienten stark belasten. Er lässt sich in gewünschter Menge in ein System zur Hämodialyse integrieren, ohne die Funktion zu beeinflussen, d.h. idealerweise auf einem non-woven-Gewebe in den Blutkappen (Fig. 2) ohne hohen Druckverlust.

Cyclodextrine sind in der Pharmazie akzeptierte, bekannte Substanzen und werden zur Herstellung von Arzneistoffen (Injektionen, Tabletten und Salben) als "Hilfsstoff" verwendet, um wasserunlösliche Medikamente zu lösen und somit biologisch verfügbar zu machen.

Durch die chemischen Eigenschaften von modifiziertem Poly-(cyclodextrin) kann bei geeigneter Präparation eine im Vergleich zu Aktivkohle um das 15 bis 200-fach erhöhte Geschwindigkeit bei der Adsorption hydrophober Toxine in wässrigen Systemen erzielt werden [23, 27].

Es werden ferner keine lebenswichtigen Mineralien, Aminosäuren oder Vitamine aus dem Blut entfernt.

Diese Erfindung beschreibt somit einen neuen Adsorber zur effektiven Entfernung von proteingebundenen Bluttoxinen bis 500 g/mol als Unterstützung einer konventionellen Hämodialyse. Er ist auf Basis von modifiziertem Cyclodextrin aufgebaut und kann mit Hilfe der LbL-Technologie an jeder beliebigen Stelle eines Systems zur Hämodialyse integriert werden.

### Literatur

[1] Vanholder R, De Smet R, Hsu C, Vogcleere P, Ringoir S. Uraemic toxicity: the middle molecule hypothesis revisited. Semin Nephrol 1994; 14: 205-218
[2] Bergström J. Uraemic toxicity. In: Kopple JD, Massry SG (eds). Nutritional Management and Renal Disease. Williams et. Wilkins, Baltimore, 1997; 97-190
[3] Hoenich NA, Woffindin C, Matthews JNS, Goldfinch ME. Turnbull J. Clinical comparison of high-flux cellulose acetate and synthetic membranes. Nephrol Dial Transplant 1994; 9: 60-66
[4] Jindal KK, McDougall J, Woods B, Nowakowski L, Goldstein MB. A study of the basic principles determining the performance of several high flux dialyzers. Am J Kidney Dis 1989; 14: 507-511
[5] Vanholder R, De Smet R, Ringoir S. Assessment of urea and other uremic markers for quantification of dialysis efficacy. Clin Chem 1992; 38: 1429-1436
[6] Niwa T. Organic acids and the uremic syndrome: protein metabolite hypothesis in the progression of chronic renal failure. Semin Nephrol 1996; 16:167-182
[7] DD 109 363, Von einem Polymer umschlossene Aktivkohle, Sparks et al.
[8] WO 2015 136107A1, Adsorbergranulat zur Entfernung urämischer Toxine, Tschulena et al.
[9] Pavlenko D, van Gereffen, van Steenbergen MJ, Glorieux G, Vanholder R, Gerritesen KGF, Stamatialis DF. New low-flux mixed matrix membranes that offer superior removal of protein-bound toxines from human plasma. Nature, Scientific Reports 2016; DOI: 10.1038/srep34429: 1-9
[10] Tijink MSL, Wester M, Sun J, Saris A, Bolhuis-Versteeg LAM, Saiful S, Joles JA, Borneman Z, Wessling M, Stamatialis DF. A novel approach for blood purification: Mixed-matrix membranes combining diffusion and adsorption in one step. Acta Biomaterialia 2012; 8: 2279-2287
[11]
   a: Glorieux et al., Uremic toxins and new methods to control their accumulation: game changers for the concept of dialysis adequacy, Clinicla Kidney Jornal 2015; 8: 353-362
   b: Krieter et al., Pilot trial on ionic strength hemodiafiltration, a novel dialysis technique for increased protein bound toxin removal, Poster EDTA 2014
[12] Dissertation Falko Böhring, Entwicklung klinischer Methoden zur vermehrten Abtrennung proteingebundener Urämie-Toxine im Rahmen einer extrakorporalen Therapie, Berlin 2013
[13] EP 0 129 905 A2, Poröse synthetische Sorbentien mit biokompatibler Oberfläche und ihre Herstellung. Tlustakova et al.
[14] Produktinformation Cytosorb, Fa. CytoSorbens Inc. NJ 08852 USA, 2012
[15] WO 2015/091842 A2, Method for removing protein-bound uremic toxines by adsorption to auxiliary substances that can be subjected to dialysis, Fislage et al.
[16] Smith RC, Riollano M, Leung A, Hammond PT. Layer-by-Layer Platform Technology for Small-Molecule Delivery. Angew. Chem. Int. Ed. 2009; 48: 8974-8977
[17] Dissertation Nina Zeh, In-vitro Versuche zur Entfernbarkeit von Furansäure, Indoxylsulfat und Pentosidin im Plasma von chronischen Hämodialysepatienten mittels Albumindialyse, Berlin 2009
[18] Dissertation Carotin Thiele, Synthese von Cycloxextrin- und Stärkederivaten zum verbesserten Wirkstofftransport, Saarbrücken 2010
[19] Tetta C, Cavaillon JM, Schulze M, Ronco C, Ghezzi PM, Camussi G, Serra AM, Lonnemann FC et G. Removal of cytokines and activated complement components in an experimental model of continuous plasma filtration coupled with sorbent adsorption. Nephrol Dial Transplant 1998; 13: 1458-1464
[20] Pedehontaa-Hiaa G, Guerrouache M, Carbonnier B, Le Derf F, Morin CJ. Layer-by-Layer Assemblies Based on a Cationic β-Cyclodextrin Polymer: Chiral Stationary Phases for Open-Tubular Electrochromatography. Chromatographia 2015; 78: 533-541
[21] Chen P, Wang X, Dong Y, Hu X. Development of a Layer-by-Layer Assembled Film on Hydrogel for Ocular Drug Delivery. Int J of Polymer Sc 2015; Article ID 535092 (9 pages)
[22] Lv S, Gao R, Cao Q, Li D, Duan J. Preparation and characterization of poly-carbaxymethyl-β-cyclodextrin superplasticizer. Cement and Concrete Research 2012; 42:1356-1361
[23] Alsbaiee A, Smith BJ, Xiao L, Ling Y, Helbling DE, Dichtel WR. Rapid removal of organic micropollutants from water by a porus β-cyclodextrin polymer. Nature 2016; 529: 790-794 / 206
[24] Jicsinszky L, Caporaso M, Tuza K, Martina K, Gaudino EC, Cravotto G. Nucleophilic Substitutions of 6 0-Monotosyl-β-cyclodextrin in a Planetary Ball Mill. Sustainable Chem Eng 2016; 4:919-929
[25] Menuel S, Doumert B, Saitzek S, Ponchel A. Delevoye L, Monlier E, Hapiot F. Selective Secondary Face Modification of Cyclodextrins by Mechanosynthesis. J Org Chem 2015; 80:6259-6266
[26] Shaw PE, Buslig BS. Selective Removal of Bitter Compounds from Grapefruit Juice and from Aqueous Solution with Cyclodextrin Polymers and with Amberlite XAD-4. J Agric Food Chem 1986; 34: 837-840
[27] Zheng Y, Wyman IW. Supramolecular Nanostructures Based on Cyclodextrin an Poly(ethylene oxide): Synthesis, Structural Characterizations and Applications for Drug Delivery. Polymers 2016; 8: 198 (18 pages)

## Patentansprüche

1. Hämokompatibler Adsorber zur Trennung von in Blut oder Blutplasma eines Patienten enthaltenen proteingebundenen Urämietoxinen mit einer Molekularmasse von < 500 g/mol, bevorzugt p-Kresol, Indoxylsulfat, Phenol, Phenolderivate, Homocystein, Urofuransäuren, insbesondere 3-Carboxy-4-methyl-5-propyl-2-furanpropionsäure, Hippursäure und p-Hydroxyhippursäure, von ihren Trägerproteinen, bevorzugt humanem Albumin,
**dadurch gekennzeichnet, dass**
der hämokompatible Adsorber ein Polymer auf Basis eines cyclischen Oligosaccharids enthält, welches auf einer festen Trägerkomponente in wenigstens einer Schicht angeordnet ist, und
das Polymer auf Basis eines cyclischen Oligosaccharids ausgewählt ist aus der Gruppe, bestehend aus: Poly(carboxymethyl-β-cyclodextrin) und Poly(trimethylammonium-β-cyclodextrin) und mit Epichlorhydrin kondensierten Cyclodextrinen, und
die feste Trägerkomponente ein poröses Material ist auf Basis von Polyethersulfon [PES], Polysulfon [PSU], Polyetheretherketone [PEEK], Polyphenylsulfon [PPSU], Polyoxymethylen [POM], Polyphenol, Polyamide, insbesondere Nylon, Polystyrol, Polycarbonat oder Polymeren, die Acrylnitril oder ein Methylallylsulfonat-Salz oder Copolymere daraus enthalten.

2. Hämokompatibler Adsorber nach einem der vorhergehenden Ansprüche, **dadurch gekennzeichnet, dass** das Polymer auf Basis eines cyclischen Oligosaccharids durch eine Layer-by-Layer-Technik in einer Vielzahl von Schichten auf der festen Trägerkomponente aufgebracht ist.

3. Separate Kartusche, die einem Dialysator eines Hämodialysesystems vorgeschaltet sein kann, ausgebildet aus einem hämokompatiblen Adsorber nach einem der Ansprüche 1 oder 2, wobei eine in der Kartusche angeordnete feste Trägerkomponente mit dem Polymer beschichtet ist.

4. Blutkappe eines Dialysators eines Hämodialysesystems, **dadurch gekennzeichnet, dass** in der Blutkappe der hämokompatible Adsorber nach einem der Ansprüche 1 oder 2 als feste Trägerkomponente, welche mit dem Polymer beschichtet ist, integriert ist.

5. Dialysator eines Hämodialysesystems, **dadurch gekennzeichnet, dass** in das Porensystem der Hohlfasermembran des Dialysators ein hämokompatibler Adsorber nach einem der Ansprüche 1 oder 2 integriert ist.

6. Vorrichtung zur Hämodiafiltration mit einem extrakorporalen Kreislauf zur Aufnahme von zu reinigendem Blut sowie mit einem Hämodialysator , der mit dem Blutkreislauf eines Patienten in Verbindung stehen kann;
wobei ein hämokompatibler Adsorber zur Trennung von in Blut oder Blutplasma eines Patienten enthaltenen proteingebundenen Urämietoxinen mit einer Molekularmasse von < 500 g/mol von ihren Trägerproteinen vorgesehen ist; und wobei
der hämokompatible Adsorber, welcher blutseitig innerhalb des Hämodialysators auf einer festen Trägerkomponente in wenigstens einer Schicht angeordnet ist, ein Adsorber gemäß den Ansprüchen 1 oder 2 ist.

## Claims

1. A hemocompatible adsorber for the separation of protein-bound uremic toxins contained in the blood or blood plasma of a patient and having a molecular mass of < 500 g/mol, preferably p-cresol, indoxyl sulfate, phenol, phenol derivatives, homocysteine, urofuranic acids, in particular 3-carboxy-4-methyl-5-propyl-2-furanopropionic acid, hippuric acid and p-hydroxyhippuric acid regarding their carrier proteins, preferably human albumin,
**characterized in that**
said hemocompatible adsorber comprises a polymer based on a cyclic oligosaccharide disposed on a solid carrier component in at least one layer, and
the polymer based on a cyclic oligosaccharide is selected from the group consisting of: poly(carboxymethyl-β-cyclodextrin) and poly(trimethylammonium-β-cyclodextrin) and cyclodextrins fused with epichlorohydrin, and
the solid carrier component is a porous material based on polyether sulfone [PES], polysulfone [PSU], polyether ether ketones [PEEK], polyphenylsulfone [PPSU], polyoxymethylene [POM], polyphenol, polyamides, in particular nylon, polystyrene, polycarbonate or polymers containing acrylonitrile or a methylallylsulfonate salt or copolymers thereof.

2. The hemocompatible adsorber according to claim 1, **characterized in that** the polymer based on a cyclic oligosaccharide is applied to the solid carrier component by a layer-by-layer technique in a plurality of layers.

3. A separate cartridge which may be arranged upstream of a dialyzer of a hemodialysis system and which is made of a hemocompatible adsorber according to one of claims 1 or 2, wherein a solid carrier component disposed in the cartridge is coated with the polymer.

4. A blood cap of a dialyzer of a hemodialysis system, **characterized in that** the hemocompatible adsorber according to one of claims 1 or 2 as a solid carrier component coated with the polymer is integrated in the blood cap.

5. A dialyzer of a hemodialysis system, **characterized in that** the hemocompatible adsorber according to one of claims 1 or 2 is integrated into the pore system of the hollow fiber membrane of the dialyzer.

6. A hemodiafiltration apparatus comprising an extracorporeal circuit for receiving blood to be purified and a hemodialyzer which may be connected to the blood circulation of a patient;
wherein a hemocompatible adsorber is provided for the separation of protein-bound uremic toxins contained in the blood or blood plasma of a patient and having a molecular mass of < 500 g/mol regarding their carrier proteins; and wherein
the hemocompatible adsorber, which is disposed on a solid carrier component in at least one layer on the blood side within the hemodialyzer, is an adsober according to claim 1 or 2.

## Revendications

1. Adsorbeur hémocompatible pour la séparation de toxines urémiques liées aux protéines contenues dans le sang ou le plasma sanguin d'un patient avec une masse moléculaire < 500 g/mol, de préférence p-crésol, sulfate d'indoxyle, phénol, dérivés phénoliques, homocystéine, acides urofuraniques, en particulier acide 3-carboxy-4-méthyl-5-propyl-2-furanpropionique, acide hippurique et acides p-hydroxyhippuriques, de leurs protéines porteuses, de préférence de l'albumine humaine,
**caractérisé en ce que**
l'adsorbeur hémocompatible contient un polymère sur la base d'un oligosaccharide cyclique, lequel est disposé sur un composant support solide selon au moins une étape, et
le polymère est choisi sur la base d'un oligosaccharide cyclique parmi le groupe consistant en : poly(carboxyméthyl-β-cyclodextrine) et poly(triméthylammonium-β-cyclodextrine) et des cyclodextrines condensées avec de l'épichlorhydrine, et
le composant support solide est un matériau poreux sur la base de polyéthersulfone [PES], polysulfone [PSU], polyétheréthercétone [PEEK], polyphénylsulfone [PPSU], polyoxyméthylène [POM], polyphénol, polyamide, en particulier nylon, polystyrène, polycarbonate ou polymères, qui contiennent de l'acrylonitrile ou un sel de méthylallylsulfonate ou des copolymères de ceux-ci.

2. Adsorbeur hémocompatible selon l'une quelconque des revendications précédentes, **caractérisé en ce que** le polymère est appliqué sur la base d'un oligosaccharide cyclique par une technique de couche par couche dans une pluralité de couches sur le composant support solide.

3. Cartouche distincte, qui peut être montée en amont d'un dialyseur d'un système d'hémodialyse, formée à partir d'un adsorbeur hémocompatible selon l'une quelconque des revendications 1 ou 2, dans laquelle l'un des composants supports solides disposés dans la cartouche est revêtu avec le polymère.

4. Bouchon de sang d'un dialyseur d'un système d'hémodialyse, **caractérisé en ce que** l'adsorbeur hémocompatible est intégré dans le bouchon de sang selon l'une quelconque des revendications 1 ou 2 en tant que composant support solide, lequel est revêtu avec le polymère.

5. Dialyseur d'un système d'hémodialyse, **caractérisé en ce qu'**un adsorbeur hémocompatible est intégré dans le système de pores de la membrane à fibres creuses du dialyseur selon l'une quelconque des revendications 1 ou 2.

6. Dispositif d'hémodiafiltration comprenant une circulation extracorporelle pour recevoir du sang à purifier ainsi qu'un hémodialyseur, qui peut être relié à la circulation extracorporelle d'un patient ;
dans lequel un adsorbeur hémocompatible pour la séparation de toxines urémiques liées aux protéines contenues dans le sang ou le plasma sanguin d'un patient est prévu avec une masse moléculaire < 500 g/mol de leurs protéines porteuses ; et dans lequel
l'adsorbeur hémocompatible, lequel est disposé côté sang dans l'hémodialyseur sur un composant support solide dans au moins une couche, est un adsorbeur selon les revendications 1 ou 2.
